# EUROPÄISCHE PATENTSCHRIFT

(11) **EP 3 285 697 B1**
(45) Veröffentlichungstag und Bekanntmachung des Hinweises auf die Patenterteilung: **17.07.2019**
(21) Anmeldenummer: 16716594.3
(22) Anmeldetag: 15.04.2016
(51) Int. Cl.: A61F 2/64, A61F 2/68

(54) **VERFAHREN ZUR STEUERUNG DER STANDPHASENDÄMPFUNG EINES KÜNSTLICHEN KNIEGELENKS**
METHOD FOR CONTROLLING THE STANDING-PHASE DAMPING OF AN ARTIFICIAL KNEE JOINT
PROCÉDÉ POUR CONTRÔLER L'AMORTISSEMENT DE LA PHASE D'APPUI D'UNE PROTHÈSE DU GENOU

(30) Priorität: 24.04.2015 DE 102015106392
(43) Veröffentlichungstag der Anmeldung: 28.02.2018
(73) Patentinhaber: Otto Bock Healthcare Products GmbH, 1070 Wien (AT)
(72) Erfinder: SEIFERT, Dirk, 1070 Wien (AT); ZARLING, Sven, 37115 Duderstadt (DE)
(74) Vertreter: Lins, Martina
(86) Internationale Anmeldenummer: PCT/EP2016/058394
(87) Internationale Veröffentlichungsnummer: WO 2016/169853

(56) Entgegenhaltungen:
- DE-A1-102006 021 802
- DE-A1-102007 053 389
- DE-A1-102008 008 284
- DE-A1-102009 052 887
- DE-A1-102009 052 890
- DE-A1-102009 052 895
- DE-A1-102012 003 369
- DE-A1-102012 013 141
- US-A1- 2014 379 096

## Beschreibung

Die Erfindung betrifft ein Verfahren zur Steuerung der Standphasendämpfung eines künstlichen Kniegelenks mit einem Oberteil und einem Unterteil, die schwenkbar um eine Schwenkachse aneinander befestigt sind, und einer Widerstandseinheit, die zwischen dem Oberteil und dem Unterteil angeordnet ist und eine Verstelleinrichtung aufweist, über die der Dämpfungswiderstand verändert werden kann, mit einer Steuereinheit, die mit der Verstelleinrichtung gekoppelt ist und die zumindest mit einem Sensor verbunden ist, wobei die Verstellung auf der Grundlage von Sensordaten erfolgt.

Kniegelenke für Orthesen, Exoskelette oder Prothesen weisen ein Oberteil mit einem oberen Anschlussteil und einem Unterteil mit einem unteren Anschlussteil auf, die gelenkig miteinander verbunden sind. An dem oberen Anschlussteil sind in der Regel Aufnahmen für einen Oberschenkelstumpf oder eine Oberschenkelschiene angeordnet, während an dem unteren Anschlussteil ein Unterschenkelrohr oder eine Unterschenkelschiene angeordnet sind. Im einfachsten Fall sind das Oberteil und das Unterteil durch ein einachsiges Gelenk verschwenkbar miteinander verbunden.

Um unterschiedliche Anforderungen während der verschiedenen Phasen eines Schrittes oder bei anderen Bewegungen oder Verrichtungen möglichst natürlich erfüllen zu können oder zu unterstützen, ist häufig eine Widerstandseinrichtung vorgesehen, die einen Flexionswiderstand und einen Extensionswiderstand bereitstellt. Über den Flexionswiderstand wird eingestellt, wie leicht sich das Unterteil im Verhältnis zum Oberteil bei einer aufgebrachten Kraft nach hinten schwingen lässt. Der Extensionswiderstand bremst die Vorwärtsbewegung des Unterteils und bildet unter anderem einen Streckanschlag aus.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssenor, ein Neigungssenor und/oder ein Kraftsensor. In Abhängigkeit von den Sensordaten wird die Position des Extensionsanschlages ermittelt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für ein Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2009 052 887 A1 beschreibt unter anderem ein Verfahren zur Steuerung eines orthetischen oder prothetischen Gelenkes mit einer Widerstandseinrichtung und Sensoren, wobei über Sensoren während der Benutzung des Gelenkes Zustandsinformationen bereitgestellt werden. Die Sensoren erfassen Momente oder Kräfte, wobei die Sensordaten von zumindest zwei der ermittelten Größen durch eine mathematische Operation miteinander verknüpft werden und dadurch eine Hilfsvariable errechnet wird, die der Steuerung des Beuge- und/oder Streckwiderstandes zugrunde gelegt wird.

Die DE 10 2012 013 141 A1 betrifft ein Verfahren zur Steuerung einer orthetischen oder prothetischen Kniegelenkseinrichtung mit einer Antriebseinrichtung zur Beaufschlagung der Kniegelenkseinrichtung mit einem Moment. Zur Unterstützung des Patienten ist es vorgesehen, dass vor der Einbeugung oder der Streckung der Kniegelenkseinrichtung dieses mit einem Beugemoment und einem Streckmoment beaufschlagt wird, das unterhalb des Niveaus liegt, was zu einer Einbeugung oder Streckung führt.

Zur Steuerung der Veränderung des Dämpfungsverhaltens werden gemäß dem Stand der Technik die Sensordaten quantitativ ausgewertet, das heißt, dass in der Regel bestimmte Grenzwerte vorgegeben werden, bei deren Erreichen oder Nichterreichen der Aktuator aktiviert oder deaktiviert wird, so dass die Widerstandseinrichtung einen erhöhten oder verringerten Flexions- oder Extensionswiderstand bereitstellt.

Patienten können Prothesen, Exoskelette oder Orthesen in verschiedenen Umgebungen einsetzen. Sie können Treppen abwärts gehen, Rampen abwärts gehen oder mit verschiedenen Geschwindigkeiten in der Ebene gehen. Weiterhin können Lasten getragen werden, was sich ebenfalls auf das Verhalten der Prothese oder Orthese auswirkt. Insbesondere nach Beendigung der Schwungphase, also nach dem Aufsetzen des versorgten Beines, wenn das Körpergewicht auf das versorgte Bein verlagert wird, besteht für die Patienten vielfach das Bedürfnis nach erhöhter Sicherheit. Eine zu hohe initiale Flexionsdämpfung, also eine Dämpfung, die einer Einbeugung des künstlichen Kniegelenkes entgegenwirkt, würde aber zu einer stoßartigen Belastung in dem Hüftgelenk führen, was eine Verringerung des Tragekomforts und der Akzeptanz der Prothese oder Orthese zur Folge hätte.

Aufgabe der vorliegenden Erfindung ist es, ein Verfahren zur Steuerung eines künstlichen Kniegelenkes, insbesondere der Dämpfungsveränderung innerhalb eines künstlichen Kniegelenkes, bereitzustellen, mit dem eine Anpassung an unterschiedliche Gangsituationen und ein komfortables Gangverhalten bei gleichzeitiger maximaler Sicherheit erreicht werden kann.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das Verfahren zur Steuerung der Standphasendämpfung eines künstlichen Kniegelenkes mit einem Oberteil und einem Unterteil, die schwenkbar um eine Schwenkachse aneinander befestigt sind, und einer Widerstandseinheit, die zwischen dem Oberteil und dem Unterteil angeordnet ist und eine Verstelleinrichtung aufweist, über die der Dämpfungswiderstand verändert werden kann, mit einer Steuereinheit, die mit der Verstelleinrichtung gekoppelt ist und die zumindest mit einem Sensor verbunden ist, wobei die Verstellung auf der Grundlage von Sensordaten erfolgt, sieht vor, dass während der Standphasenbeugung bis zur terminalen Standphase der Kniewinkel über den zumindest einen Sensor erfasst wird und die Flexionsdämpfung auf ein Niveau oberhalb einer initialen Flexionsdämpfung bis zur Vermeidung eines weiteren Einbeugens bei Erreichen eines festgelegten maximalen Kniewinkels angehoben wird. Nach Beendigung der Schwungphase wird der Dämpfungswiderstand, wesentlich ist der Flexionswiderstand, auf ein Niveau eingestellt, das geeignet ist, bei einem Fersenkontakt eine ausreichende Dämpfung bereitzustellen. Das Niveau ist dann das Niveau einer initialen Flexionsdämpfung. Im weiteren Verlauf der Standphase erhöht sich der Kniewinkel, da sich durch den Fersenkontakt impulsartig eine Bodenreaktionskraft aufbaut und aufgrund der Krafteinleitung zusammen mit einer Vorwärtsbewegung des Körpers ein Drehmoment um die Schwenkachse bewirkt wird. Ein unversorgtes Bein würde aufgrund von Muskelkontraktionen den Fersenstoß abfedern, bei einem versorgten Bein wird dies durch die Widerstandseinheit mit der initialen Flexionsdämpfung bewirkt. Beim initialen Bodenkontakt oder Fersenstoß wird ein Flexionswinkel oder Kniewinkel von ungefähr 5° erreicht. Bei der nächsten Bewegungsphase, der Belastungsantwort, verstärkt sich die Belastung des Beines mit dem Körpergewicht, was zu einer weiteren Einbeugung oder Destabilisierung des Kniegelenkes führt. Ein unversorgtes Bein wird eine weitere Einbeugung durch Anspannung der Beinstreckermuskulatur abfangen. Erfindungsgemäß ist vorgesehen, dass nach dem initialen Bodenkontakt oder Fersenstoß die Flexionsdämpfung auf ein Niveau oberhalb der initialen Flexionsdämpfung angehoben wird, das ausreicht, um ein weiteres Einbeugen bei Erreichen eines festgelegten maximalen Kniewinkels zu unterbinden. Der maximal erreichbare Kniewinkel wird vorab festgelegt, beim Gehen auf Rampen oder in der Ebene liegt der maximale Kniewinkel in einem Bereich zwischen 7° und 12°. Die Veränderung der Flexionsdämpfung erfolgt somit dahingehend, dass die Flexionsdämpfung bei zunehmendem Kniewinkel, wobei eine maximal extendierte Stellung den Kniewinkel 0° hat, überwacht wird, ob und wie sich der Kniewinkel vergrößert. Nähert sich der Kniewinkel dem festgelegten maximalen Kniewinkel an, wird die Flexionsdämpfung erhöht, bevorzugt findet eine progressive Erhöhung statt, also zunächst eine langsame Erhöhung der Flexionsdämpfung, um dann kurz vor Erreichen des festgelegten maximalen Kniewinkels die Flexionsdämpfung so weit anzuheben, dass eine weitere Einbeugung nicht stattfinden kann, mithin ein Flexionsstop bewirkt wird. Die Widerstandseinheit, die beispielsweise als hydraulische, pneumatische, elektrische oder mechanische Widerstandseinheit oder Bremse ausgestaltet sein kann, wird blockiert. Bei hydraulischen oder pneumatischen Widerstandseinheiten werden Überströmkanäle geschlossen, so dass kein Medium mehr aus einer Extensionskammer in eine Flexionskammer strömen kann. Bei mechanischen Widerstandseinrichtungen wird beispielsweise die Reibung so weit erhöht, dass keine weitere Flexion stattfinden kann, Gleiches gilt für elektrisch aktuierte Widerstandseinheiten. Das Verfahren ist zur Steuerung sowohl von Prothesen als auch von Orthesen und Exoskeletten vorgesehen. Wird nachfolgend von Orthesen gesprochen, so gelten die Ausführungen ebenso für die Sonderform der Orthese in Gestalt eines Exoskelettes. Der Sensor oder die Sensoren erfassen die Messgrößen über die gesamte Standphase, als von dem Moment des ersten Kontaktes des Fußes oder Fußteiles mit dem Boden, dem initialen Bodenkontakt, Fersenstoß oder Heel Strike, bis zur terminalen Standphase, in der eine vollständige Streckung des Kniegelenkes erreicht ist und das Kniegelenk über einen Widerstand gegen eine Dorsalextension und die Position des Kraftvektors vor der Knieachse in extendierter Stellung gehalten wird. Nach der terminalen Standphase, in der sogenannten Vor-Schwungphase, wird die Schwungphase vorbereitet, bei einem gesunden Bein tritt eine passive Flexion des Kniegelenkes ein, die Zehen sind jedoch noch nicht vom Boden abgehoben.

Eine Weiterbildung der Erfindung sieht vor, dass die Flexionsdämpfung auf dem Niveau gehalten wird, das bei Erreichen des maximalen Kniewinkels anliegt. Dadurch wird ein erhöhtes Maß an Sicherheit bereitgestellt, so dass das künstliche Kniegelenk im weiteren Gangverlauf nicht kollabiert. Beim Gehen in der Ebene folgt nach einem relativen Maximum ein Abfallen des Kniewinkels, also eine Extension, die bis zur termialen Standphase reicht. In dieser Phase verringert sich der Kniewinkel bis zu einer vollständigen Streckung. Die Aufrechterhaltung der Flexionsdämpfung auf dem Niveau des maximalen Kniewinkels während der Belastungsantwort stellt somit eine Vergrößerung der Sicherheit dar, ohne dass das Gehen in der Ebene negativ beeinflusst werden würde.

Die Flexionsdämpfung kann während des initialen Fersenkontaktes konstant gehalten und in Abhängigkeit von der Belastung erhöht werden. Die Erhöhung erfolgt vorteilhafterweise erst nach Erreichen der sogenannten Belastungsantwort, einer Phase des Ganges, in der der Fuß vollständigen Bodenkontakt hat und der Kraftvektor nach dem initialen Fersenstoß hinter die Knieachse wandert, wodurch die Kniebeugung bewirkt und bei einem gesunden Bein der vordere Oberschenkelmuskel aktiviert wird, um die Einbeugung abzubremsen. Bei dem künstlichen Kniegelenk wird die Flexionsdämpfung in Abhängigkeit von der Belastung die Flexionsdämpfung nach dem Heel Strike erhöht, bei einer großen Belastung mehr erhöht als bei einer geringen Belastung.

Eine Weiterbildung der Erfindung sieht vor, dass eine Vorwärtsrotation des Unterteils, also des Unterschenkelrohres bei einer Prothese oder bei einer Orthese einer Unterschenkelschiene, um einen distalen Drehpunkt erfasst wird, bevorzugt sensorisch erfasst wird. Es wird das Bewegen des Unterschenkels um das Knöchelgelenk oder um einen fiktiven Drehpunkt im Bodenbereich ermittelt oder detektiert, um Rückschlüsse über das weitere Bewegungsverhalten ziehen zu können. Bei einer fortgesetzten Vorwärtsrotation nach Erreichen des maximalen Kniewinkels kann dann die Flexionsdämpfung verringert werden, um während der terminalen Standphase oder der Vorschwungphase ein ausreichend leichtes Einbeugen des Kniegelenkes zu ermöglichen, damit eine Zehenablösung einfach stattfinden kann. Eine fortwährende Vorwärtsrotation auch bei einem Nichtabfall des Kniewinkels kann beispielsweise beim Abwärtsgehen auf Rampen und Treppen erfolgen, wenn eine weitere Vorwärtsrotation des Unterteils durch das weitere Abrollen um die Ferse oder um den Drehpunkt auf einer Kante einer Treppenstufe vorliegt, was z.B. durch einen separaten Sensor, beispielsweise einem Inertialwinkelsensor, detektiert werden kann. Auch dieser Sensor ist mit der Steuereinheit verbunden, der mit der Verstelleinrichtung gekoppelt ist und den Flexionswiderstand anpasst.

Die Flexionsdämpfung kann nach Überschreiten eines festgelegten Bereiches der Vorwärtsrotation und/oder bei einem sich verringernden Kniewinkel verringert werden. Der Bereich der Vorwärtsrotation kann beispielsweise einen festgelegten Winkelbereich umfassen, um den das Unterteil eine Vorwärtsdrehung um einen distalen Drehpunkt herum ausführen muss. Werden beispielsweise 5° bis 10° Vorwärtsrotation detektiert, nimmt die Steuerung an, dass entweder beim Gehen in der Ebene oder bei einem alternierenden Abwärtsgehen auf Rampen und/oder Treppen eine weitere Vorwärtsbewegung stattgefunden hat und eine Verringerung der Flexionsdämpfung notwendig ist. Die Verringerung der Flexionsdämpfung auf der Grundlage der Überwachung eines festgelegten Bereiches einer Vorwärtsrotation kann separat oder in Kombination mit der Detektion eines sich verringernden Kniewinkels eingesetzt werden.

Die Flexionsdämpfung kann nach dem Einstellen der maximalen Flexionsdämpfung der anfänglichen Standphase auf einen Wert oberhalb oder gleich der initialen Standphasenflexionsdämpfung verringert werden. Dadurch wird beispielsweise beim Abwärtsgehen von Rampen oder Treppen auch ein erhöhtes Sicherheitsniveau gegen ungewolltes Einbeugen bereitgestellt.

Eine Weiterbildung der Erfindung sieht vor, dass nach Erreichen eines festgelegten maximalen Kniewinkels und einer Vorwärtsrotation des Unterteils die Flexionsdämpfung verringert wird. Wird beim Gehen, insbesondere beim Abwärtsgehen von Rampen oder schrägen Ebenen, der maximale Kniewinkel erreicht, der für den Patienten ermittelt oder vorgegeben wird, und erfolgt darüber hinaus eine Vorwärtsrotation des Unterteils, die beispielsweise durch einen Inertialwinkelsensor oder einen Knöchelgelenkswinkelsensor detektiert wird, wird erfindungsgemäß der Flexionswiderstand verringert, um ein weiteres Einbeugen des Kniegelenkes und damit ein gleichmäßiges Gehen und ein natürliches Gangbild bereitzustellen.

Der maximale Kniewinkel kann aus einem Bereich zwischen 7° und 12° oder aus einem Bereich zwischen 11° und 9° ausgewählt und festgelegt werden, ein häufiger Wert für maximale Kniewinkel liegt bei 10°. Dies ist ungefähr die Hälfte desjenigen Kniewinkels, der von einem unversorgten Bein als Einbeugung während der Belastungsantwort erreicht wird.

Der maximale Kniewinkel kann auch auf der Grundlage statistischer Auswertungen erfasster Kniewinkel für das Gehen in der Ebene festgelegt werden, wobei die statistischen Auswertungen in der Steuereinheit des künstlichen Kniegelenkes durchgeführt werden können. Dadurch wird ermöglicht, dass der maximale Kniewinkel für den jeweiligen Nutzer des künstlichen Kniegelenkes individuell festlegbar ist.

Das Verfahren ist zur Steuerung der Standphasendämpfung insbesondere beim Gehen in der Ebene sowie beim Rampen abwärtsgehen geeignet, die Sondersituation eines Treppabgehens erfordert eine andere Steuerung.

Die Widerstandseinheit kann beispielsweise als Aktuator, beispielsweise als hydraulische, pneumatische, magnetorheologische, magnetische, elektrische, mechanische oder elektromagnetische Widerstandseinheit ausgestaltet sein. Bei hydraulischen oder pneumatischen Widerstandseinheiten werden Überströmkanäle geschlossen, so dass diese Überströmkanäle kein Medium mehr aus einer Extensionskammer in eine Flexionskammer strömen kann. Auf diese Weise kann der Fluss des Mediums zwischen der Extensionskammer und der Flexionskammer ggf. auch vollständig unterbunden werden. Bei mechanischen Widerstandseinrichtungen wird beispielsweise die Reibung soweit erhöht, dass keine weitere Flexion stattfinden kann. Gleiches gilt für elektrisch aktuierte Widerstandseinheiten.

Es können auch Aktuatoren zum Einsatz kommen, die sowohl Energie aktiv in das System einbringen, als auch umgekehrt dem System Energie entziehen und auf diese Weise als Widerstandseinheit wirken. Aktuatoren können beispielsweise als Elektromotoren, hydraulische oder pneumatische Pumpen oder piezoelektrische Elemente ausgebildet sein.

Nachfolgend wird ein Ausführungsbeispiel der Erfindung anhand der beigefügten Figuren näher erläutert. Es zeigen:
- Figur 1 -: eine schematische Darstellung einer Beinprothese; sowie
- Figur 2 -: eine Darstellung eines Steuerungsdiagramms.

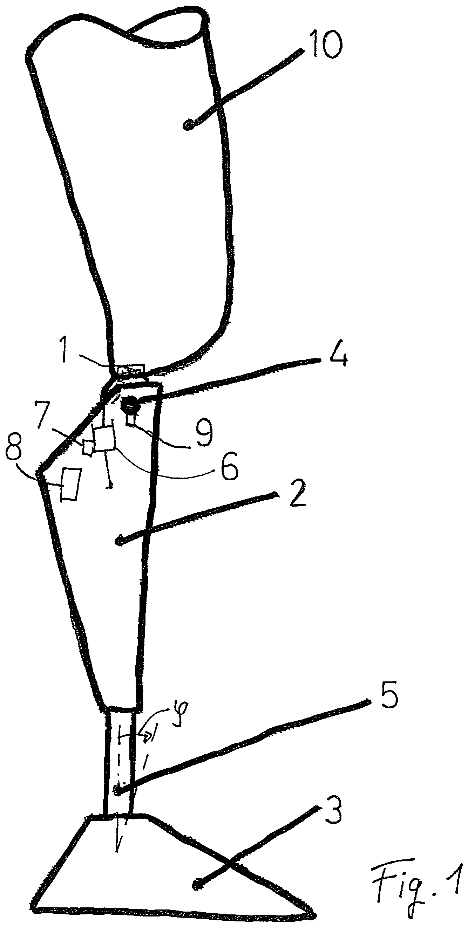

In der Figur 1 ist in einer schematischen Darstellung eine Beinprothese mit einem Oberteil 1 abgebildet, an dem ein Oberschenkelschaft 10 zur Aufnahme eines Oberschenkelstumpfes befestigt ist. An dem Oberteil 1 ist ein Unterteil 2 in Gestalt eines Unterschenkelteils schwenkbar angeordnet. Das Unterteil 2 ist an dem Oberteil 1 um eine Schwenkachse 4 schwenkbar gelagert. Das Unterteil 2 weist ein Unterschenkelrohr 5 auf, an dessen distalen Ende ein Prothesenfuß 3 befestigt ist, in dem eine Einrichtung zur Ermittlung der wirksamen Axialkraft auf das Unterschenkelrohr 5 sowie des Knöchelmomentes, das um die Befestigungsstelle des Prothesenfußes 3 an dem Unterschenkelrohr 5 wirksam ist, untergebracht sein kann.

In oder an dem Unterteil 2 ist eine Widerstandseinrichtung 6, die beispielsweise als Dämpfer oder Aktuator ausgebildet sein kann, angeordnet, die sich zwischen dem Oberteil 1 und dem Unterteil 2 abstützt, um einen einstellbaren Extensionswiderstand und Flexionswiderstand bereitzustellen. Der Widerstandseinrichtung 6 ist eine Verstelleinrichtung 7 zugeordnet, beispielsweise ein Motor, ein Magnet oder ein anderer Aktuator, über den der jeweilige Widerstand R innerhalb der Widerstandseinrichtung 6 verändert werden kann. Ist die Widerstandseinrichtung 6 als Hydraulikdämpfer oder Pneumatikdämpfer ausgebildet, kann über die Verstelleinrichtung 7 der jeweilige Strömungsquerschnitt eines Überströmkanals vergrößert oder verkleinert werden. Ebenso kann durch die Verstelleinrichtung 7 der Strömungswiderstand auf eine andere Art und Weise variiert werden. Dies kann beispielsweise durch Öffnen oder Schließen von Ventilen oder Veränderungen von Viskositäten oder magnetorheologischer Eigenschaften geschehen. Ist die Widerstandseinrichtung als ein Elektromotor im Generatorbetrieb ausgebildet, kann durch Veränderung des elektrischen Widerstandes eine Vergrößerung oder Verringerung der jeweiligen Widerstände gegen eine Flexion oder Extension eingestellt werden.

Um die Verstelleinrichtung 7 aktivieren oder deaktivieren zu können, ist eine Steuerungseinrichtung 8 dem Unterteil 2 zugeordnet, insbesondere in einer Unterschenkelverkleidung angebracht, über die ein entsprechendes Aktivierungs- oder Deaktivierungssignal an die Verstelleinrichtung 7 abgegeben wird. Die Verstelleinrichtung 7 wird auf der Basis von Sensordaten aktiviert oder deaktiviert, die Sensordaten werden von einem oder mehreren Sensoren 9 geliefert, die an dem künstlichen Kniegelenk angeordnet sind. Dies können Winkelsensoren, Beschleunigungssensoren und/oder Kraftsensoren sein. Die Sensoren 9 sind mit der Steuereinrichtung 8 verbunden, beispielsweise per Kabel oder über eine drahtlose Sendeeinrichtung. In dem dargestellten Ausführungsbeispiel ist der Sensor 9 unter anderem als Kniewinkelsensor ausgebildet.

Über die Sensoren 9 wird der gesamte Schrittzyklus von dem Fersenstoß (Heel Strike) bis zum erneuten, nachfolgenden Heel Strike HS überwacht, somit auch die gesamte Schwungphase mit der Schwungphasenextension und der Schwungphasenflexion.

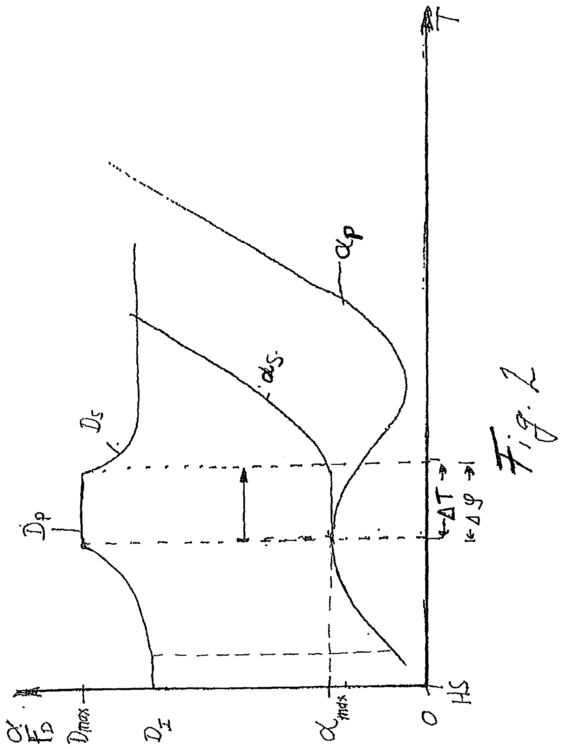

In der Figur 2 ist in einem Diagramm über die Zeit der Kniewinkel α sowie die Flexionsdämpfung F_{D} aufgetragen. Der Kniewinkel α ist für zwei Gehsituationen von Beginn der Standphase, also vom Heel Strike HS, bis zur terminalen Standphase skizziert. Der untere Kurvenverlauf α_{P} zeigt den Kniewinkelverlauf für das Gehen in der Ebene, der obere Kurvenverlauf α_{S} zeigt den Kniewinkelverlauf für das Abwärtsgehen auf einer Rampe oder Treppe, wobei es sich hier um die Darstellung des versorgten Beines bei einem alternierenden Gehen handelt.

Der übliche Kniewinkelverlauf für das Gehen in der Ebene gemäß Kurve α_{P} beginnt bei einer maximal extendierten Stellung im Bereich des Heel Strike HS, führt nach dem initialen Bodenkontakt zu einem Ansteigen des Kniewinkels α bis zu einem lokalen Maximum αₘₐₓ am Ende der Belastungsantwort, um dann während der mittleren Standphase wieder abzufallen. Das Bein befindet sich dann in einer nahezu gestreckten Stellung, im weiteren Verlauf des Schrittes steigt der Kniewinkel α im Bereich der terminalen Standphase und Vorschwungphase wieder an.

Der Kniewinkelverlauf für das alternierende Abwärtsgehen auf Rampen, wie in der Kurve α_{S} dargestellt, hat keinen Abfall am Ende der Belastungsantwort, vielmehr verbleibt das künstliche Kniegelenk auf einem konstanten Wert αₘₐₓ, bis es dann früher als beim Gehen in der Ebene weiter eingebeugt wird.

Um für beide Gangmuster ein sicheres Dämpfungsverhalten der Standhasenbeugung bereitzustellen, wird der Dämpfungswiderstand F_{D} oder Flexionswiderstand zunächst auf ein initiales Dämpfungsniveau F_{DI} eingestellt, das eine Einbeugung des künstlichen Kniegelenkes beim initialen Bodenkontakt erlaubt, die Einbeugung jedoch dämpft und abbremst, um ein Kollabieren des künstlichen Kniegelenkes zu vermeiden. Diese initiale Flexionsdämpfung F_{DI} wird zunächst konstant beibehalten, bis der Kniewinkel α einen Schwellwert erreicht hat. In dem dargestellten Ausführungsbeispiel beträgt der Schwellwert ca. 30% des festgelegten, zulässigen oder für zulässig erachteten maximalen Kniewinkels αₘₐₓ, die Erhöhung der Flexionsdämpfung F_{D} beginnt nahezu unmittelbar nach dem Fersenkontakt. Alternativ kann der Schwellwert auch bei einem größeren Kniewinkel liegen, beispielsweise bei 50% oder 70% des festgelegten, zulässigen oder für zulässig erachteten maximalen Kniewinkels αₘₐₓ. Bei Erreichen des festgelegten Schwellwertes für den Kniewinkel α wird die Flexionsdämpfung F_{D} erhöht, um die weitere Einbeugung des Kniegelenkes zu bremsen und bei Erreichen des maximalen Kniewinkels αₘₐₓ zu sperren. Im dargestellten Ausführungsbeispiel wird die Flexionsdämpfung progressiv erhöht, sie kann aber auch degressiv oder linear erhöht werden. Die Flexionsdämpfung F_{D} befindet sich bei Erreichen des maximalen Kniewinkels αₘₐₓ auf dem maximalen Flexionsdämpfungswert F_{Dmax}, bei dem eine weitere Flexion nicht mehr möglich ist. Dieser Widerstandswert F_{Dmax} wird über einen festgelegten Zeitraum ΔT gehalten, es bildet sich ein Plateau Dp des maximalen Flexionswiderstandes F_{Dmax} aus, während dieser Zeit ist keine Flexion des künstlichen Kniegelenkes möglich.

Der Zeitraum ΔT, für den dieses Niveau F_{Dmax} gehalten wird, wird entweder über ein Zeitschaltglied oder über die Erfassung einer Vorwärtsrotation Δϕ des Unterteils 2, beispielsweise des Unterschenkelrohres 5 oder eine Verschwenkung um das Knöchelgelenk des Prothesenfußes 3, detektiert. Findet eine weitere Vorwärtsrotation um den Winkel ϕ statt, die durch Beschleunigungssensoren, Winkelsensoren und/oder Inertialwinkelsensoren erfasst werden kann, bewegt sich der Nutzer des künstlichen Kniegelenkes weiter nach vorne. Als möglicher Winkelbereich für eine weitere Vorwärtsrotation ϕ können 5° bis 10° Verschwenkwinkel angenommen werden. Nach Erreichen des Grenzwertes für die weitere Vorwärtsrotation oder des Zeitablaufes wird die Flexionsdämpfung F_{Dmax} verringert. Die Verringerung erfolgt im dargestellten Ausführungsbeispiel degressiv, so dass zunächst ein schneller Abfall der Flexionsdämpfung bewirkt wird, beispielsweise um eine weitere Einbeugung beim Rampengehen, wie es der Verlauf der Kurve α_{S} zeigt, zu ermöglichen. Andere Verläufe der Dämpfungsverringerung können eingestellt werden, beispielsweise progressiv oder linear. In dem Plateaubereich mit dem Widerstandsplateau Dp während der Zeit ΔT findet keine weitere Flexionsbewegung des künstlichen Kniegelenkes statt. Erst nach Absenken des Flexionswiderstandes F_{D} auf ein Niveau unterhalb des maximalen Flexionswiderstandes F_{Dmax}, im dargestellten Ausführungsbeispiel oberhalb eines Niveaus der initialen Flexionsdämpfung F_{DI} im Bereich der Absenkkurve F_{DS}, wird eine zunehmende Einbeugung ermöglicht.

Der Vorteil dieser Steuerung liegt in dem hohen Maß an Sicherheit, die durch eine anfängliche, stets limitierte Kniebeugung auf den maximalen Kniewinkel αₘₐₓ begründet ist, ohne dass dabei der Bewegungsumfang oder die Funktionalität während des weiteren Bewegungsablaufes eingeschränkt ist. Wird der maximale Kniewinkel αₘₐₓ erreicht und rotiert das Bein weiter nach vorne, ohne dass es zu einer Kniestreckung kommt, wie in der Kurve α_{S} gezeigt, wird die Flexionsdämpfung F_{D} kontinuierlich bis auf eine hohe Dämpfung, gegebenenfalls bis auf die initiale Flexionsdämpfung F_{DI} reduziert. Somit wird bei dem Abwärtsgehen auf Rampen oder Treppen ohne Sicherheitseinbußen sichergestellt, dass ein ungestörter weiterer Bewegungsablauf stattfinden kann.

## Patentansprüche

1. Verfahren zur Steuerung der Standphasendämpfung eines künstlichen Kniegelenkes mit einem Oberteil (1) und einem Unterteil (2), die schwenkbar um eine Schwenkachse (4) aneinander befestigt sind, und einer Widerstandseinheit (6), die zwischen dem Oberteil (1) und dem Unterteil (2) angeordnet ist und eine Verstelleinrichtung (7) aufweist, über die der Dämpfungswiderstand (F_{D}) verändert werden kann, mit einer Steuereinheit (8), die mit der Verstelleinrichtung (7) gekoppelt ist und die zumindest mit einem Sensor (9) verbunden ist, wobei die Verstellung auf der Grundlage von Sensordaten erfolgt, **dadurch gekennzeichnet, dass** während der Standphasenbeugung bis zur terminalen Standphase der Kniewinkel (a) über den zumindest einen Sensor (9) erfasst wird und die Flexionsdämpfung (F_{D}) auf ein Niveau (F_{Dmax}) oberhalb einer initialen Flexionsdämpfung (F_{DI}) bis zur Vermeidung eines weiteren Einbeugens bei Erreichen eines festgelegten maximalen Kniewinkels (αₘₐₓ) angehoben wird.

2. Verfahren nach Anspruch 1, **dadurch gekennzeichnet, dass** die Flexionsdämpfung (F_{D}) auf dem Niveau (F_{Dmax)} bei Erreichen des maximalen Kniewinkels (αₘₐₓ) gehalten wird.

3. Verfahren nach Anspruch 1 oder 2, **dadurch gekennzeichnet, dass** die Flexionsdämpfung (F_{D}) während des initialen Fersenkontaktes konstant gehalten und in Abhängigkeit von der Belastung die Flexionsdämpfung (F_{D}) erhöht wird.

4. Verfahren nach einem der voranstehenden Ansprüche, **dadurch gekennzeichnet, dass** eine Vorwärtsrotation des Unterteils (2) um einen distalen Drehpunkt erfasst wird und bei einer fortgesetzten Vorwärtsrotation nach Erreichen des maximalen Kniewinkels (αₘₐₓ) die Flexionsdämpfung (F_{D}) verringert wird.

5. Verfahren nach einem der voranstehenden Ansprüche, **dadurch gekennzeichnet, dass** die Flexionsdämpfung (F_{D}) nach Überschreiten eines festgelegten Bereiches der Vorwärtsrotation und/oder bei einem sich verringernden Kniewinkel (α) verringert wird.

6. Verfahren nach Anspruch 4 oder 5, **dadurch gekennzeichnet, dass** die Flexionsdämpfung (F_{D}) auf einen Wert größer oder gleich der initialen Standphasenflexionsdämpfung (F_{DI}) verringert wird.

7. Verfahren nach einem der voranstehenden Ansprüche, **dadurch gekennzeichnet, dass** nach Erreichen eines festgelegten maximalen Kniewinkels (α) und einer relativen Vorwärtsrotation des Unterteils (2) die Flexionsdämpfung (F_{D}) verringert wird.

8. Verfahren nach einem der voranstehenden Ansprüche, **dadurch gekennzeichnet, dass** der maximale Kniewinkel (αₘₐₓ) aus einem Bereich zwischen 7° und 12° oder aus einem Bereich zwischen 9° und 11° ausgewählt und festgelegt wird.

9. Verfahren nach einem der voranstehenden Ansprüche, **dadurch gekennzeichnet, dass** der maximale Kniewinkel (αₘₐₓ) auf der Grundlage statistischer Auswertungen erfasster Kniewinkel (α) für das Gehen in der Ebene festgelegt wird.

10. Verfahren nach einem der voranstehenden Ansprüche, **dadurch gekennzeichnet, dass** der maximale Kniewinkel (αₘₐₓ) für den jeweiligen Nutzer des künstlichen Kniegelenkes individuell festlegbar ist.

## Claims

1. A method for controlling the stance phase damping of an artificial knee joint having an upper part (1) and a lower part (2) which are fastened to one another in a manner pivotable about a pivot axis (4) and having a resistance unit (6) which is arranged between the upper part (1) and the lower part (2) and which has an adjustment device (7) by means of which the damping resistance (F_{D}) can be modified, having a control unit (8) which is coupled to the adjustment device (7) and which is connected at least to a sensor (9), wherein the adjustment is carried out on the basis of sensor data, **characterized in that**, during the stance phase flexion up until the terminal stance phase, the knee angle (a) is detected by means of the at least one sensor (9), and the flexion damping (F_{D}) is increased to a level (F_{Dmax)} above an initial flexion damping (F_{DI}), to the point of prevention of further flexion when a set maximum knee angle (αₘₐₓ) is reached.

2. The method as claimed in claim 1, **characterized in that** the flexion damping (F_{D}) is held at the level (F_{Dmax}) when the maximum knee angle (αₘₐₓ) is reached.

3. The method as claimed in claim 1 or 2, **characterized in that** the flexion damping (F_{D}) is held constant during the initial heel strike and the flexion damping (F_{D}) is increased in a manner dependent on the load.

4. The method as claimed in one of the preceding claims, **characterized in that** a forward rotation of the lower part (2) about a distal center of rotation is detected and, in the case of a continued forward rotation after the maximum knee angle (αₘₐₓ) is reached, the flexion damping (F_{D}) is decreased.

5. The method as claimed in one of the preceding claims, **characterized in that** the flexion damping (F_{D}) is decreased after an overshooting of a set range of the forward rotation and/or in the case of a decreasing knee angle (α).

6. The method as claimed in claim 4 or 5, **characterized in that** the flexion damping (F_{D}) is decreased to a value greater than or equal to the initial stance phase flexion damping (F_{DI}).

7. The method as claimed in one of the preceding claims, **characterized in that** the flexion damping (F_{D}) is decreased after a set maximum knee angle (α) is reached and after a relative forward rotation of the lower part (2).

8. The method as claimed in one of the preceding claims, **characterized in that** the maximum knee angle (αₘₐₓ) is selected and set from a range between 7° and 12° or from a range between 9° and 11°.

9. The method as claimed in one of the preceding claims, **characterized in that** the maximum knee angle (αₘₐₓ) is defined on the basis of statistical evaluations of detected knee angles (α) for walking on a level surface.

10. The method as claimed in one of the preceding claims, **characterized in that** the maximum knee angle (αₘₐₓ) can be individually set for the respective user of the artificial knee joint.

## Revendications

1. Procédé pour commander l'amortissement en phase debout d'une articulation artificielle de genou, comportant une partie supérieure (1) et une partie inférieure (2) qui sont fixées l'une à l'autre de façon mobile en basculement autour d'un axe de basculement (4), et une unité de résistance (6) qui est agencée entre la partie supérieure (1) et la partie inférieure (2) et qui présente un moyen de réglage (7) permettant de modifier la résistance d'amortissement (F_{D}), comportant une unité de commande (8) qui est couplée au moyen de réglage (7) et qui est connectée au moins à un capteur (9), le réglage ayant lieu en se basant sur des données de capteur,
**caractérisé en ce que**
pendant la flexion en phase debout jusqu'à la phase debout terminale, l'angle (α) du genou est détecté par ledit au moins un capteur (9) et l'amortissement de flexion (F_{D}) est augmenté à un niveau (F_{Dmax)} au-dessus d'un amortissement de flexion initial (F_{DI}) jusqu'à éviter une poursuite de la flexion lorsqu'un angle maximal fixé (αₘₐₓ) du genou est atteint.

2. Procédé selon la revendication 1,
**caractérisé en ce que**
l'amortissement de flexion (F_{D}) est maintenu au niveau (F_{Dmax)} lorsque l'angle maximal (αₘₐₓ) du genou est atteint.

3. Procédé selon la revendication 1 ou 2,
**caractérisé en ce que**
l'amortissement de flexion (F_{D}) est maintenu constant pendant le contact initial du talon, et l'amortissement de flexion (F_{D}) est augmenté en fonction de la charge.

4. Procédé selon l'une des revendications précédentes,
**caractérisé en ce que**
une rotation vers l'avant de la partie inférieure (2) autour d'un point de rotation distal est détectée et lors d'une poursuite de la rotation vers l'avant, une fois que l'angle maximal (αₘₐₓ) du genou est atteint, l'amortissement de flexion (F_{D}) est réduit.

5. Procédé selon l'une des revendications précédentes,
**caractérisé en ce que**
l'amortissement de flexion (F_{D}) est réduit après dépassement d'une zone fixée de la rotation vers l'avant et/ou lors d'une réduction de l'angle (α) du genou.

6. Procédé selon la revendication 4 ou 5,
**caractérisé en ce que**
l'amortissement de flexion (F_{D}) est réduit à une valeur supérieure ou égale à l'amortissement de flexion initial (F_{DI}) en phase debout.

7. Procédé selon l'une des revendications précédentes,
**caractérisé en ce que**
l'amortissement de flexion (F_{D}) est réduit une fois que l'angle maximal fixé (α) du genou est atteint et après une rotation relative vers l'avant de la partie inférieure (2).

8. Procédé selon l'une des revendications précédentes,
**caractérisé en ce que**
l'angle maximal (αₘₐₓ) du genou est choisi et fixé dans une plage entre 7° et 12° ou dans une plage entre 9° et 11°.

9. Procédé selon l'une des revendications précédentes,
**caractérisé en ce que**
l'angle maximal (αₘₐₓ) du genou est fixé en se basant sur des évaluations statistiques d'angles de genou (α) saisis pour la marche dans le plan.

10. Procédé selon l'une des revendications précédentes,
**caractérisé en ce que**
l'angle maximal (αₘₐₓ) du genou peut être fixé individuellement pour l'utilisateur respectif de l'articulation artificielle de genou.
